# EUROPEAN PATENT SPECIFICATION

(11) **EP 3 752 132 B1**
(45) Date of publication and mention of the grant of the patent: **08.12.2021**
(21) Application number: 19703995.1
(22) Date of filing: 07.02.2019
(51) Int. Cl.: A61K 9/14, A61K 9/19, A61K 31/506, A61P 35/00

(54) **PHARMACEUTICAL COMPOSITION OF BRIGATINIB**
PHARMAZEUTISCHE ZUSAMMENSETZUNGEN AUS BRIGATINIB
COMPOSITION PHARMACEUTIQUE DE BRIGATINIB

(30) Priority: 13.02.2018 EP 18156509; 22.02.2018 EP 18158053; 13.09.2018 EP 18194237
(43) Date of publication of application: 23.12.2020
(73) Proprietor: Sandoz AG, 4056 Basel (CH)
(72) Inventor: MARTIN, Nolwenn, 6250 Kundl (AT)
(74) Representative: Prüfer & Partner mbB Patentanwälte · Rechtsanwälte
(86) International application number: PCT/EP2019/052969
(87) International publication number: WO 2019/158421

(56) References cited:
- EP-A1- 2 000 139
- WO-A1-2016/065028
- SERAJUDDIN ET AL: "SOLID DISPERSION OF POORLY WATER-SOUBLE DRUGS: EARLY PROMISES, SUBSEQUENT PROBLEMS, AND RECENT BREAKTHROUGHS", JOURNAL OF PHARMACEUTICAL SCIENCES, AMERICAN PHARMACEUTICAL ASSOCIATION, US, vol. 88, no. 10, 1 October 1999 (1999-10-01), pages 1058-1066, XP000851882, ISSN: 0022-3549, DOI: 10.1021/JS980403L

## Description

### FIELD OF THE INVENTION

The present invention relates to a solid dispersion comprising amorphous brigatinib and methods for preparing the same. The invention also concerns a pharmaceutical composition comprising an effective amount of a solid dispersion comprising amorphous brigatinib and at least one further pharmaceutically acceptable excipient, as well as methods for its preparation. The pharmaceutical composition of the present invention may be used as a medicament in particular for the treatment of anaplastic lymphoma kinase (ALK) positive metastatic non-small cell lung cancer (NSCLC) and other diseases.

### BACKGROUND OF THE INVENTION

Brigatinib known under the chemical name 5-chloro-*N*4-[2-(dimethylphosphoryl)phenyl]-*N*2-[2-methoxy-4-[4-(4-methylpiperazin-1-yl)piperidin-1-yl]phenyl]pyrimidine-2,4-diamine can be represented by the following chemical structure according to Formula (A):

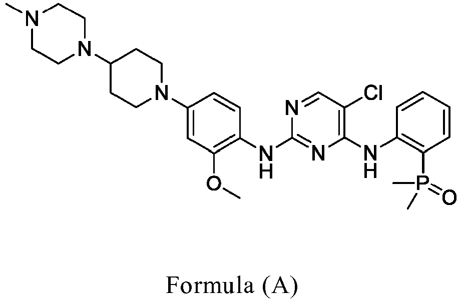

Brigatinib, previously also known as AP26113, is a potent multi-targeted tyrosine-kinase inhibitor used in the treatment of non-small cell lung cancer (NSCLC) and other diseases. It acts as both as anaplastic lymphoma kinase (ALK) and epidermal growth factor receptor (EGFR) inhibitor.

Brigatinib and its synthesis are described in WO 2009/143389 A1. In example 122 brigatinib is isolated after chromatographic purification, followed by an additional work-up consisting of suspending the yellow solid obtained after chromatography in ethyl acetate and refluxing the suspension for 30 minutes, cooling it to room temperature and filtering it. The obtained solid is then dissolved in dichloromethane, filtered and evaporated to afford brigatinib as an off-white solid. The physical form of brigatinib obtained from this example is not described.

WO 2016/065028 A1 discloses crystalline brigatinib and more particularly the crystalline forms of brigatinib designated as form A, form B, form C, form D, form E, form F, form G, form H, form J, form K and form L. Form A is described as an anhydrous and non-hygroscopic form. Form B is reported as a hydroscopic form and is obtained by dehydration of form C or form D. Form B reversibly convents to the hydrated crystalline forms C or D at relative humidity above 30% or transforms to form A, e.g. at elevated temperature (above 150 °C). The crystalline forms E to H are solvated forms, which convert to form A upon storage under atmospheric conditions. The crystalline forms J to L are obtained in mixtures containing form J or form K or form L together with form A.

In WO 2016/065028 A1, Table 27 summarizes the results of over 600 experiments and shows that most of the experiments (i.e. 98% of the experiments) led to the formation of crystalline form A of brigatinib as a pure form or in a mixture containing form A and one or two additional forms of brigatinib. Brigatinib form A is described as the most stable form of brigatinib and indeed form A is used as the active pharmaceutical ingredient in the marketed product "Alunbrig", which was approved by the US FDA in April 2017.

WO 2016/065028 A1 describes amorphous brigatinib in three experiments and uses amorphous brigatinib as starting material for the polymorph screening experiments, but not for the preparation of pharmaceutical formulations.

In general, amorphous forms are thermodynamically metastable with respect to the crystalline state. Above their glass transition temperatures (Tg) amorphous materials show a high tendency towards crystallization. Crystallization is often accelerated by temperature as well as by water adsorption because the plasticizing effect of water results in a decrease of the glass transition temperature.

While amorphous solids have increased solubility and an accelerated dissolution rate in comparison to their crystalline counterparts, crystalline solid forms of an active pharmaceutical ingredient are typically preferred for the preparation of pharmaceutical compositions, such as tablets, for various reasons such as stability with respect to the solid form or chemical stability or ease of handling during formulation of a pharmaceutical.

As mentioned above, tablets comprising the crystalline form A of brigatinib have been approved by the US FDA for the treatment of ALK-positive metastatic non-small cell lung cancer (NSCLC). While the 30mg tablets have an approved shelf-life of 24 months, the 90mg tablets have interestingly been approved only with a shelf life of 18 months based on the provided stability data (Center for Drug Evaluation and Research, Approval Package for application number 208772Orig1s000, page 2).

### SUMMARY OF THE INVENTION

The present inventors have surprisingly found that the solid dispersions of the present invention comprising amorphous brigatinib have a higher thermal stability than the marketed brigatinib form A. The present inventors have managed to prepare stable pharmaceutical compositions comprising amorphous brigatinib despite brigatinib's high tendency to crystallize. The pharmaceutical compositions of the present invention are advantageous compared to pharmaceutical compositions comprising crystalline brigatinib form A in that their higher thermal stability can improve storage stability, for example under high temperature conditions. One objective of the present invention is thus to provide a pharmaceutical composition comprising an effective amount of amorphous brigatinib which retains amorphous character and/or chemical stability upon storage, for example throughout its shelf-life.

Brigatinib has a high tendency to crystallize, as known from WO 2016/065028 A1 where in one experiment upon lyophilisation crystalline brigatinib form A was obtained. The present inventors surprisingly found that amorphous brigatinib can be stabilized in the context of a solid dispersion further comprising at least one pharmaceutically acceptable excipient, wherein the at least one pharmaceutically acceptable excipient is (i) hydroxypropylmethylcellulose (HPMC), and/or (ii) a silicon-based inorganic adsorbent having a BET specific surface area of at least 1 m²/g and preferably having a pH of at least 5.5 as determined using a pH meter in a solution of 400 mg of the silicon-based inorganic compound in 10 mL of de-ionized water at 25 °C. Even more surprisingly, the solid dispersion comprising amorphous brigatinib was found to demonstrate higher thermal and moisture stability than crystalline brigatinib form A.

Therefore, the present invention relates to solid dispersions of amorphous brigatinib with (i) HPMC and/or (ii) specific silica carriers, and to methods for their preparation. The invention also relates to a pharmaceutical composition comprising an effective amount of a solid dispersion comprising amorphous brigatinib, and to a method for the preparation thereof.

### Definitions

As used herein the term "room temperature" refers to a temperature in the range of from 20 to 30 °C, preferably to a temperature in the range of from 22 to 27 °C, more preferably to a temperature in the range of from 23 to 26 °C.

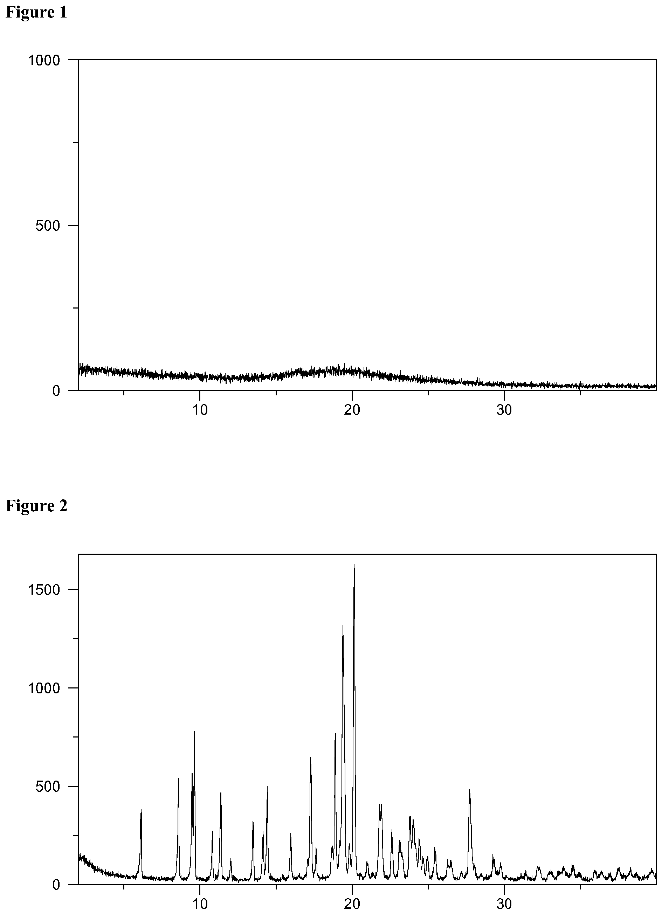

As used herein, the term "amorphous" is used for non-crystalline material, which lacks long-range inter-molecular order. Amorphous solids are generally isotopic, i.e. exhibit similar properties in all directions. When analyzed by powder X-ray diffraction, no sharp characteristic peaks are observed, but instead one or several broad peaks (e.g. "halos") appear. Figure 1 is a typical example of a powder X-ray diffraction pattern of an amorphous solid.

The term "solid dispersion" as used herein refers to a system in a solid state (as opposed to a liquid or gaseous state) wherein one component is dispersed more or less evenly throughout the other component or components, e.g. in the context of the present invention amorphous brigatinib within the pharmaceutically acceptable excipient, wherein the at least one pharmaceutically acceptable excipient is (i) an organic polymer, and/or (ii) a silicon-based inorganic adsorbent.

As used herein the term "glass transition temperature" corresponds to a change in physical state from a glassy (rigid, highly viscous, brittle) material to a more fluid or malleable rubbery state ("Polymorphism in the Pharmaceutical Industry", Chapter 10.4.1 "The amorphous state" Petit S., Coquerel G., Wiley edited by Hilfiker R.).

The term "reflection" with regards to powder X-ray diffraction as used herein, means peaks in an X-ray diffractogram, which are caused at certain diffraction angles (Bragg angles) by constructive interference from X-rays scattered by parallel planes of atoms in solid material, which are distributed in an ordered and repetitive pattern in a long-range positional order. Such a solid material is classified as crystalline material, whereas amorphous material is defined as solid material, which lacks long-range order and only displays short-range order (also see explanation above), thus resulting in broad scattering. According to literature, long-range order e.g. extends over approximately more than 100 atoms, whereas short-range order is over a few atoms only (see *"*Fundamentals of Powder Diffraction and Structural Characterization of Materials" by Vitalij K. Pecharsky and Peter Y. Zavalij, Kluwer Academic Publishers, 2003, page 3).

In the context of the present invention, a reference to powder X-ray diffraction patterns means that they were measured / are to be measured at a temperature in the range of from 20 to 30 °C with Cu-Kalpha_{1,2} radiation having a wavelength of 0.15419 nm.

A solid dispersion comprising amorphous brigatinib and at least one pharmaceutically acceptable excipient, wherein the at least one pharmaceutically acceptable excipient is (i) HPMC, and/or (ii) a specific silicon-based inorganic adsorbent, may be referred herein as being characterized by graphical data "as shown in" a figure. Such data include, for example, powder X-ray diffractograms. The person skilled in the art understands that factors such as variations in instrument type, response and variations in sample directionality, sample concentration and sample purity may lead to small variations for such data when presented in graphical form.

As used herein, the term "silicate" refers to naturally occurring or synthesized compounds containing an anionic silicon compound, preferably an oxide.

The term "silica" as used herein refers to silicon dioxide in its various forms, such as naturally occurring or synthesized silica.

As used herein the term "silicon-based inorganic adsorbent" refers to a silicon-based inorganic compound having a high porosity and a large surface area that allows at least some adsorption of amorphous brigatinib to it. The "silicon based inorganic adsorbent" is an inert material with a sufficiently high BET specific surface area of at least 1m²/g. Preferably, the BET specific surface area is at least 10m²/g, such as from 10m²/g to 1000m²/g, for example from 20m²/g to 500m²/g, such as from 200m²/g to 450m²/g.

As used herein, the term "measured at a temperature in the range of from 20 to 30 °C" refers to a measurement under standard conditions. Typically, standard conditions mean a temperature in the range of from 20 to 30 °C, i.e. at room temperature. A preferred temperature for measurements is 22 °C. Typically, standard conditions additionally mean a measurement at 20% to 75% RH, with about 20% to 40% RH being a preferred controlled humidity value for a measurement.

As used herein, the terms "essentially pure" or "substantially pure" with reference to a physical form of brigatinib, means that the physical form includes less than about 10%, more preferably less than about 5%, even more preferably less than about 3%, and most preferably less than about 1% by weight of any other physical form of brigatinib.

The term "physical form" as used herein refers to any crystalline and amorphous phase of brigatinib.

The term "about" as used herein means within 5%, more typically within 1% and most typically within 0.5% of the indicated value or range.

A "predetermined amount" as used herein with regard to a solid dispersion comprising amorphous brigatinib and at least one pharmaceutically acceptable excipient, wherein the at least one pharmaceutically acceptable excipient is (i) an organic polymer, and/or (ii) a silicon-based inorganic adsorbent, refers to a defined amount of said solid dispersion to be used for the preparation of a unit dose of a pharmaceutical composition.

The term "effective amount" as used herein with regard to a solid dispersion comprising amorphous brigatinib and at least one pharmaceutically acceptable excipient, wherein the at least one pharmaceutically acceptable excipient is (i) an organic polymer, and/or (ii) a silicon-based inorganic adsorbent, encompasses an amount of said solid dispersion, which causes a desired therapeutic and/or prophylactic effect.

The term "polymer" as used herein is a naturally occurring or synthetic compound having a molecular weight of at least 5000 Da, wherein at least 10 repeating identical or similar subunits are linked by covalent chemical bonds. Copolymers are comprised by the term "polymer" as used herein. In an "organic polymer" the subunits (monomers) comprise at least one carbon-carbon bond.

### Abbreviations

- ALK: anaplastic lymphoma kinase
- NSCLC: non-small cell lung cancer
- PXRD: powder X-ray diffraction
- RH: relative humidity
- Tg: glass transition temperature
- TGMS: thermogravimetric mass spectroscopy
- HPMC: hydroxypropylmethylcellulose

### BRIEF DESCRIPTION OF THE DRAWINGS

- Figure 1:: Representative PXRD of amorphous brigatinib prepared according to the procedure described in Comparative Example 1 herein. The X-axis shows the 2-theta angle / °, with tick marks, from left to right, at 10, 20, 30 ° 2-theta. The Y-axis shows the intensity / counts, with tick marks, from bottom to top, at 500, 1000, 1500.
- Figure 2:: Representative PXRD of amorphous brigatinib prepared according to the procedure described in comparative example 1 herein, after 1 week at 40 °C and 75% RH. The X-axis shows the 2-theta angle / °, with tick marks, from left to right, at 10, 20, 30 ° 2-theta. The Y-axis shows the intensity / counts, with tick marks, from bottom to top, at 500, 1000.

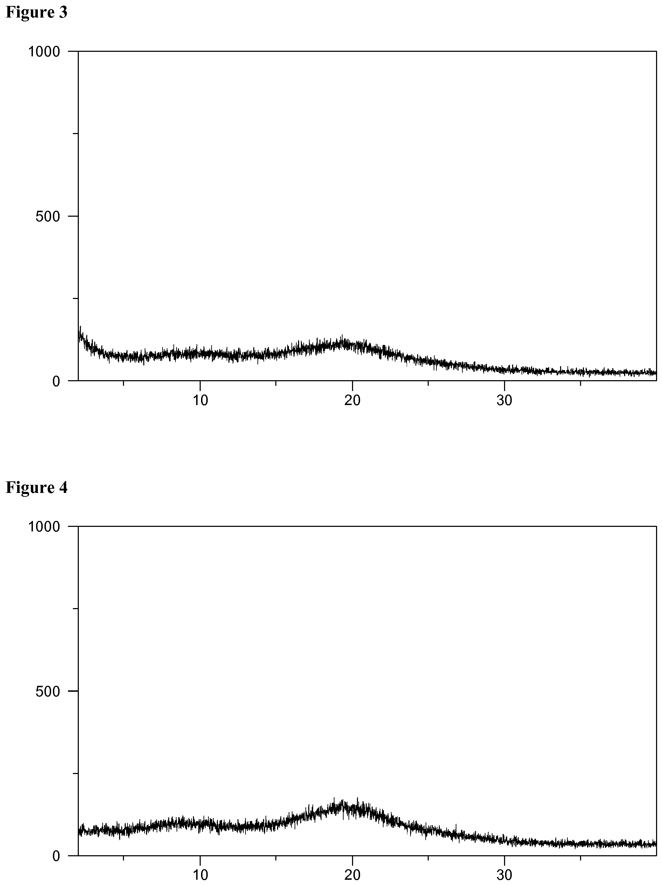

- Figure 3:: Representative PXRD of the solid dispersion comprising amorphous brigatinib and HPMC prepared according to the procedure described in Example 1 herein. The X-axis shows the 2-theta angle / °, with tick marks, from left to right, at 10, 20, 30 ° 2-theta. The Y-axis shows the intensity / counts, with tick marks, from bottom to top, at 500, 1000.
- Figure 4:: Representative PXRD of the solid dispersion comprising amorphous brigatinib and HPMC prepared according to the procedure described in Example 1 herein, after storage at 40 °C and 75% relative humidity for 1 week. The X-axis shows the 2-theta angle / °, with tick marks, from left to right, at 10, 20, 30 ° 2-theta. The Y-axis shows the intensity / counts, with tick marks, from bottom to top, at 500, 1000.

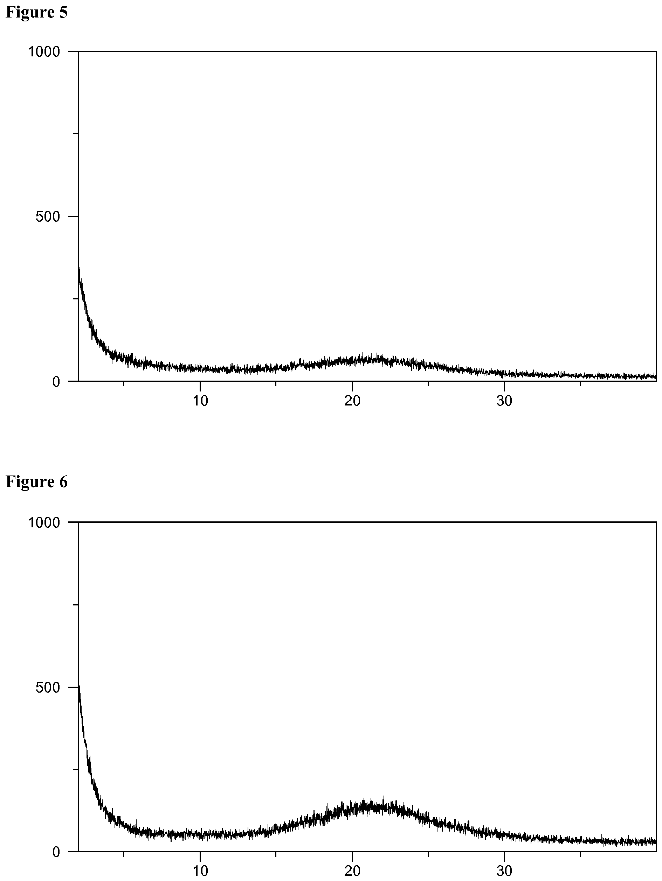

- Figure 5:: Representative PXRD of the solid dispersion comprising amorphous brigatinib and Syloid^{®} 72P prepared according to the procedure described in Example 2-1 herein. The X-axis shows the 2-theta angle / °, with tick marks, from left to right, at 10, 20, 30 ° 2-theta. The Y-axis shows the intensity / counts, with tick marks, from bottom to top, at 500, 1000.
- Figure 6:: Representative PXRD of the solid dispersion comprising amorphous brigatinib and Syloid^{®} 72P prepared according to the procedure described in Example 2-1 herein, after storage at 40 °C and 75% relative humidity for 1 week. The X-axis shows the 2-theta angle / °, with tick marks, from left to right, at 10, 20, 30 ° 2-theta. The Y-axis shows the intensity / counts, with tick marks, from bottom to top, at 500, 1000.

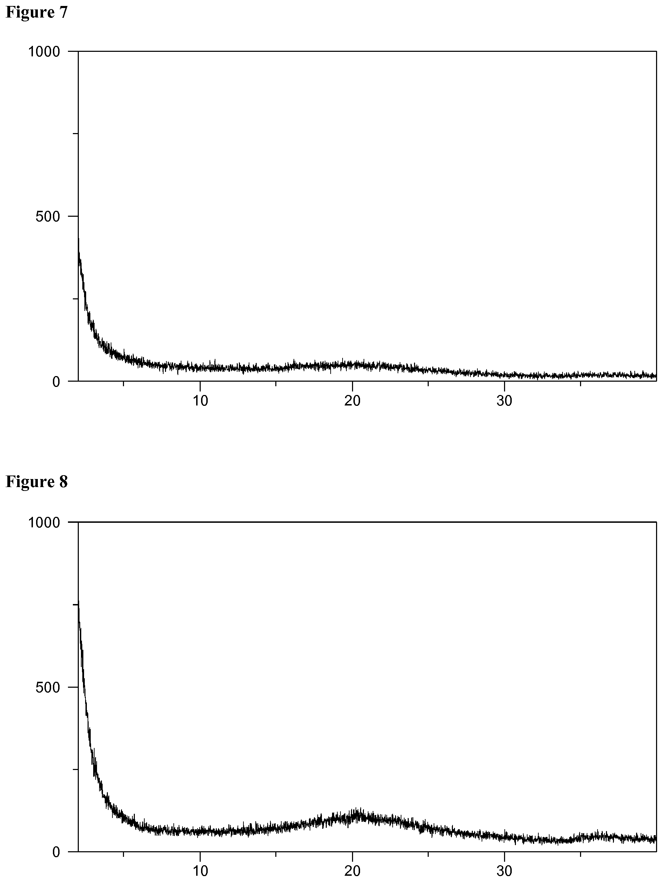

- Figure 7:: Representative PXRD of the solid dispersion comprising amorphous brigatinib and Neusilin^{®} UFL2 prepared according to the procedure described in Example 3-1 herein. The X-axis shows the 2-theta angle / °, with tick marks, from left to right, at 10, 20, 30 ° 2-theta. The Y-axis shows the intensity / counts, with tick marks, from bottom to top, at 500, 1000.
- Figure 8:: Representative PXRD of the solid dispersion comprising amorphous brigatinib and Neusilin^{®} UFL2 prepared according to the procedure described in Example 3-1 herein, after storage at 40 °C and 75% relative humidity for 1 week. The X-axis shows the 2-theta angle / °, with tick marks, from left to right, at 10, 20, 30 ° 2-theta. The Y-axis shows the intensity / counts, with tick marks, from bottom to top, at 500, 1000.

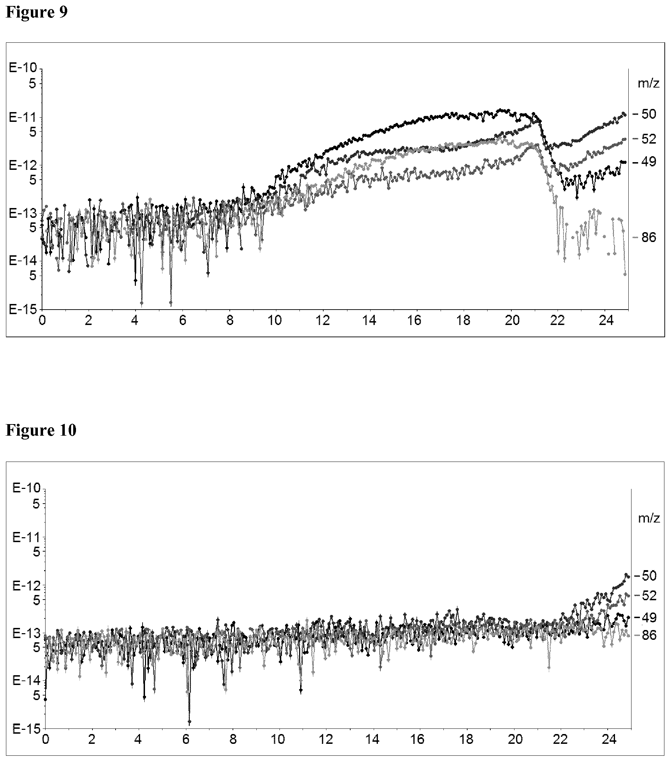

- Figure 9:: Thermogravimetric mass spectroscopy (TGMS) thermogram of brigatinib form A. The X-axis shows the time / min, which is correlated with the sample temperature (sample kept at 25°C for 2.5 min, then heated from 25°C to 250°C at a rate of 10°C/min; e.g. sample temperature: 100°C after 10 min, 200°C after 20 min). The Y-axis shows the ion current / A. "m/z" corresponds to the mass-to-charge ratio. At about 100°C the chemistry of the form A sample changes.
- Figure 10:: Thermogravimetric mass spectroscopy (TGMS) thermogram of the solid dispersion comprising amorphous brigatinib and Syloid^{®} 72P prepared according to the procedure described in Example 2-1 herein. The X-axis shows the time / min, which is correlated with the sample temperature (sample kept at 25°C for 2.5 min, then heated from 25°C to 250°C at a rate of 10°C/min; e.g. sample temperature: 100°C after 10 min, 200°C after 20 min). The Y-axis shows the ion current / A. "m/z" corresponds to the mass-to-charge ratio. The chemistry of the sample remains unchanged at temperatures where the form A sample changes its composition.

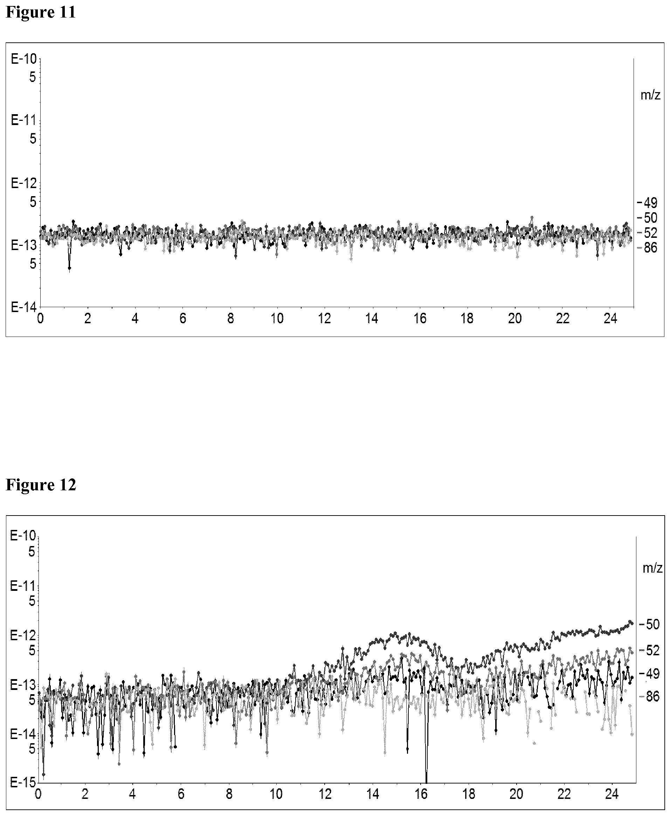

- Figure 11:: Thermogravimetric mass spectroscopy (TGMS) thermogram of the solid dispersion comprising amorphous brigatinib and Neusilin^{®} UFL2 prepared according to the procedure described in Example 3-1 herein. The X-axis shows the time / min, which is correlated with the sample temperature (sample kept at 25°C for 2.5 min, then heated from 25°C to 250°C at a rate of 10°C/min; e.g. sample temperature: 100°C after 10 min, 200°C after 20 min). The Y-axis shows the ion current / A. "m/z" corresponds to the mass-to-charge ratio. The chemistry of the sample remains unchanged at temperatures where the form A sample changes its composition.
- Figure 12:: Thermogravimetric mass spectroscopy (TGMS) thermogram of the solid dispersion comprising amorphous brigatinib and HPMC prepared according to the procedure described in Example 1 herein. The X-axis shows the time / min, which is correlated with the sample temperature (sample kept at 25°C for 2.5 min, then heated from 25°C to 250°C at a rate of 10°C/min; e.g. sample temperature: 100°C after 10 min, 200°C after 20 min). The Y-axis shows the ion current / A. "m/z" corresponds to the mass-to-charge ratio.

### DETAILED DESCRIPTION OF THE INVENTION

When the invention is described below in further detail by reference to embodiments, it is not limited thereto.

In one aspect, the present invention relates to a solid dispersion comprising amorphous brigatinib and at least one pharmaceutically acceptable excipient, wherein the at least one pharmaceutically acceptable excipient is (i) HPMC, and/or (ii) a silicon-based inorganic adsorbent having a BET specific surface area of at least 1 m²/g and preferably having a pH of at least 5.5 as determined using a pH meter in a solution of 400 mg of the silicon-based inorganic compound in 10 mL of de-ionized water at 25 °C.

For the solid dispersion of the invention comprising amorphous brigatinib and at least one pharmaceutically acceptable excipient, wherein the at least one pharmaceutically acceptable excipient is (i) HPMC, and/or (ii) a silicon-based inorganic adsorbent having a BET specific surface area of at least 1 m²/g and preferably having a pH of at least 5.5 as determined using a pH meter in a solution of 400 mg of the silicon-based inorganic compound in 10 mL of de-ionized water at 25 °C,
the weight ratio of brigatinib and the pharmaceutically acceptable excipient is from 1.0 : 0.5 to 1.0 : 10.0, more preferably from 1.0:1.0 to 1.0:8.0 such as 1.0 : 1.0 to 1.0 : 6.0, for example 1.0 : 1.0 to 1.0 : 5.0. The weight ratio is calculated based on the total amount of brigatinib present in said solid dispersion and based on the total amount of the pharmaceutically acceptable excipients present in said solid dispersion, i.e. if two or more pharmaceutically acceptable excipients are present in the solid dispersion of the present invention, the combined weight of these is taken for the calculation of the weight ratio. A preferred weight ratio is from 1.0 : 1.0 to 1.0 : 5.0.

In a further preferred embodiment, the solid dispersion of the present invention is a solid dispersion wherein amorphous brigatinib is chemically stable.

By "chemically stable" it is meant that amorphous brigatinib present in the solid dispersion of the present invention shows very little degradation when analyzed by thermogravimetric mass spectroscopy (TGMS) from 25°C to 250°C at a heating rate of 10°C/min. By "chemically stable upon storage under stress conditions" it is meant that amorphous brigatinib present in the solid dispersion of the present invention shows very little degradation upon storage under stress conditions, i.e. when stored at a relative humidity of 70 % at 40°C for 14 days, preferably after storage for 50 days. Very little degradation means that the TGMS thermogram shows no relevant decomposition process or an HPLC analysis of brigatinib shows no impurity of more than 0.1 area%.

The absence of peaks in the powder X-ray diffractogram of the solid dispersion of the invention, in particularly the absence of characteristic PXRD peaks of forms A, B, C and D of brigatinib, suggests that in the solid dispersion of the present invention brigatinib is substantially in amorphous form.

The characteristic powder X-ray diffraction pattern of brigatinib form A comprises peaks at 2-theta angles of 9.6, 17.2, 19.4, 20.1, 23.1 and 27.7°. The characteristic powder X-ray diffraction pattern of brigatinib form B comprises peaks at 2-theta angles of 11.5, 14.5, 16.9, 19.2 and 23.2°. The characteristic powder X-ray diffraction pattern of brigatinib form C comprises peaks at 2-theta angles of 5.4, 14.9, 15.9, 17.3, 19.2 and 23.9°. The characteristic powder X-ray diffraction pattern of brigatinib form D comprises peaks at 2-theta angles of 9.7, 11.1, 17.4, 18.9 and 23.7°.

The present invention addresses drawbacks of the known crystalline forms of brigatinib, i.e. forms A, B, C, D and brigatinib solvates, and in particular of the marketed form A of brigatinib, by providing a solid dispersion comprising amorphous brigatinib and at least one pharmaceutically acceptable excipient as matrix material, wherein the at least one pharmaceutically acceptable excipient is (i) HPMC, and/or (ii) a silicon-based inorganic adsorbent having a BET specific surface area of at least 1 m²/g and preferably having a pH of at least 5.5 as determined using a pH meter in a solution of 400 mg of the silicon-based inorganic compound in 10 mL of de-ionized water at 25 °C.

The brigatinib in the context of the solid dispersion of the present invention is stable in the amorphous state upon storage, as judged by the absence of characteristic PXRD peaks after storage. Amorphous brigatinib itself readily converts to crystalline form A of brigatinib upon storage for as short as one week only. Also in the context of solid dispersions with carriers such as polyvinylpyrrolidon PVP-40000 or Syloid^{®} AL-1FP, amorphous brigatinib converts to crystalline brigatinib upon storage.

"Stable in the amorphous stage upon storage" within the meaning of the present invention means that the solid dispersion of the present invention after storage at a relative humidity of 45% at 23°C for 7 days, preferably even after storage for 14 days, more preferably even after storage for 50 days, shows no signs of the presence of crystalline brigatinib as judged by the absence of peaks at 2-theta angles of 9.6, 17.2, 19.4, 20.1, 23.1 and 27.7°, or as judged by the absence of peaks at 2-theta angles of 11.5, 14.5, 16.9, 19.2 and 23.2°, or as judged by the absence of peaks at 2-theta angles of 5.4, 14.9, 15.9, 17.3, 19.2 and 23.9°. In particular the solid dispersion of the present invention after storage at a relative humidity of 45% at 23°C for 7 days shows no signs of the presence of any one of the known forms A, B, C and D of crystalline brigatinib, as judged by the absence of peaks at 2-theta angles of 9.6, 17.2, 19.4, 20.1, 23.1 and 27.7° (form A), 11.5, 14.5, 16.9, 19.2 and 23.2° (form B), 5.4, 14.9, 15.9, 17.3, 19.2 and 23.9° (form C), and 9.7, 11.1, 17.4, 18.9 and 23.7° (form D) in an powder X-ray diffractogram (PXRD).

"Stable in the amorphous state upon storage under stress conditions" in the context of the present invention means that the solid dispersion of the present invention when stored at a relative humidity of 70 % at 40 °C for 7 days, such as after storage for 14 days, more preferably after storage for 50 days, shows no signs of the presence of crystalline brigatinib as judged by the absence of peaks at 2-theta angles of 9.6, 17.2, 19.4, 20.1, 23.1 and 27.7°, or as judged by the absence of peaks at 2-theta angles of 11.5, 14.5, 16.9, 19.2 and 23.2°, or as judged by the absence of peaks at 2-theta angles of 5.4, 14.9, 15.9, 17.3, 19.2 and 23.9°, and in particular shows no signs of the presence of any one of the known forms A, B, C and D of crystalline brigatinib, as judged by the absence of peaks at 2-theta angles of 9.6, 17.2, 19.4, 20.1, 23.1 and 27.7° (form A), 11.5, 14.5, 16.9, 19.2 and 23.2° (form B), 5.4, 14.9, 15.9, 17.3, 19.2 and 23.9° (form C), and 9.7, 11.1, 17.4, 18.9 and 23.7° (form D) in an powder X-ray diffractogram (PXRD).

Thus, in a preferred embodiment, the solid dispersion of the present invention is a solid dispersion wherein the amorphous brigatinib is stable in the amorphous physical form upon storage. More preferably, the solid dispersion of the present invention is a solid dispersion wherein the amorphous brigatinib is stable in the amorphous physical form upon storage under stress conditions.

It is preferred that the solid dispersion of the invention itself is amorphous, i.e. that the solid dispersion of the present invention does not only show no sign of the presence of any crystalline brigatinib, but that the solid dispersion comprising amorphous brigatinib and at least one pharmaceutically acceptable excipient, wherein the at least one pharmaceutically acceptable excipient is (i) HPMC, and/or (ii) a silicon-based inorganic adsorbent having a BET specific surface area of at least 1 m²/g and preferably having a pH of at least 5.5 as determined using a pH meter in a solution of 400 mg of the silicon-based inorganic compound in 10 mL of de-ionized water at 25 °C, shows no characteristic peak, preferably no peak, in an PXRD. It is more preferred that the amorphous solid dispersion is stable in the amorphous physical form upon storage, and in particular upon storage under stress conditions.

In one aspect, the pharmaceutically acceptable excipient is hydroxypropylmethylcellulose (HPMC).

In another aspect, the pharmaceutically acceptable excipient is a silicon-based inorganic adsorbent having a BET specific surface area of at least 1 m²/g and preferably having a pH of at least 5.5 as determined using a pH meter in a solution of 400 mg of the silicon-based inorganic compound in 10 mL of de-ionized water at 25 °C. The silicon-based inorganic compound is preferably a silicon-based inorganic adsorbent, i.e. a silicon-based inorganic compound having a high porosity and a large surface area that enable it to adsorb brigatinib to it. The "silicon based inorganic adsorbent" is preferably a material with a high BET specific surface area of at least 1 m²/g. Preferably, the BET specific surface area is at least 10 m²/g, such as from 10 m²/g to 1000 m²/g, for example from 20 m²/g to 500 m²/g, such as from 200 m²/g to 450 m²/g. The BET specific surface area is determined using the Brunauer-Emmet-Teller method described in "The Journal of the American Chemical Society", Vol. 60, page 309, February 1938 and corresponding to the International Standard ISO 5794/1 (Appendix D). Preferred silicon-based inorganic adsorbents are silica, silicates, and combinations of one or more silica with one or more silicate(s).

Preferably, the bulk density of the silicon-based inorganic compound is in the range of from 10 to 600 g/L, such as from 20 to 500 g/L, preferably in the range of from 30 to 450 g/L, more preferably in the range of from 50 to 400 g/L, such as from 50 g/L to 250 g/L. Bulk density as used herein is defined as tapped bulk density determined according to Method A on page 4 of the WHO document QAS/11.450 FINAL from March 2012, with the heading "S.3.6. BULK DENSITY AND TAPPED DENSITY OF POWDERS". Generally, it is conceivable that the solid dispersion of the present invention contains at least one silicon-based inorganic compound having a bulk density in the above-defined preferred ranges and at least one silicon-based inorganic compound having a bulk density outside these ranges. Preferably, all silicon-based inorganic compound comprised in the solid composition of the present invention have a bulk density in the above-defined preferred ranges.

In one aspect, the silicon-based inorganic compound is silica. The silica is preferably selected from the group consisting of fumed silica, precipitated silica, gel silica, colloidal silica, and a combination of two or more thereof, such as a combination of fumed silica and precipitated silica or a combination of fumed silica and colloidal silica or a combination of fumed silica and gel silica or a combination of precipitated silica and gel silica or a combination of precipitated silica and colloidal silica or a combination of gel silica and colloidal silica or a combination of fumed silica and precipitated silica and gel silica or a combination of fumed silica and gel silica and colloidal silica or a combination of precipitated silica and gel silica and colloidal silica or a combination of fumed silica and precipitated silica and gel silica and colloidal silica. Preferred silica can be characterized by having an average particle size (Malvern laser) of from 1µm to 20µm, such as from 2µm to 10µm. Preferred silica can also be characterized by having a bulk density of from 30 g/l to 200 g/l, such as from 50 g/l to 150 g/l. More preferred silica can be characterized by having an average particle size (Malvern laser) of from 1µm to 20µm, such as from 2µm to 10µm, and by having a bulk density of from 30 g/l to 200 g/l, such as from 50 g/l to 150 g/l. The commercially available compounds Syloid^{®} 72 FP and Syloid^{®} 244 FP, all from Grace, are examples of particularly preferred silicas.

In another aspect, the silicon-based compound can be a silicate. The silicate is preferably an aluminosilicate or an aluminometasilicate which, more preferably, additionally contains at least one alkali metal element selected from the group consisting of Li, Na, K, Rb, Cs and a combination of two or more thereof, preferably from the group consisting of Li, Na, K, and a combination of two or more thereof, more preferably from the group consisting of Na, K, and a combination of two or more thereof, and/or at least one alkaline earth metal element selected from the group consisting of Mg, Ca, Sr, Ba, and a combination of two or more thereof, preferably from the group consisting of Mg, Ca, Ba, and a combination of two or more thereof, preferably from the group consisting of Mg, Ca, and a combination of two or more thereof. More preferably, the silicate is an aluminosilicate or an aluminometasilicate which additionally contains at least one alkaline earth metal element selected from the group consisting of Mg, Ca, Sr, Ba, and a combination of two or more thereof, preferably from the group consisting of Mg, Ca, Ba, and a combination of two or more thereof, preferably from the group consisting of Mg, Ca, and a combination of two or more thereof. More preferably, the silicate is an aluminometasilicate, which additionally contains Mg. Preferred magnesium aluminometasilicate include, but are not restricted to, the commercially available compounds Neusilin^{®} UFL2, Neusilin^{®} US2, both from Fuji Chemical Industry Co., Ltd.

Examples of silicates include, but are not restricted to, nesosilicates comprising the structure unit [SiO₄]₄-, sorosilicates comprising the structure unit [Si₂O₇]₆-, cyclosilicates comprising the structure unit [SiₙO₃ₙ]₂ₙ-, single chain inosilicates comprising the structure unit [SiₙO₃ₙ]₂ₙ-, double chain inosilicates comprising the structure unit [Si₄ₙO11ₙ]₆ₙ-, phyllosilicates comprising the structure unit [SiₙO₅ₙ]₂ₙ-, or tectosilicates with a 3D framework comprising the structure unit [AlₓSi_{y}O_{2(x+y)}]ₓ-.

Silicon-based inorganic compounds have preferably a pH in a defined range, preferably a pH of at least 5.5 as determined using a pH meter in a solution of 400 mg of the silicon-based inorganic compound in 10 mL of de-ionized water at 25 °C. More preferably, the at least one silicon-based compound has a pH in the range of from 5.5 to 9.0, more preferably in the range of from 6.0 to 8.5, more preferably in the range of from 6.5 to 8.0.

Generally, it is conceivable that the solid dispersion of the present invention contains at least one silicon-based inorganic compound having a pH in the above-defined preferred ranges and at least one silicon-based inorganic compound having a pH outside these ranges. Preferably, all silicon-based inorganic compounds comprised in the solid composition of the present invention have a pH in the above-defined preferred ranges.

Generally, the silica and/or the silicate can be present in crystalline or amorphous form. Preferably, at least 90 weight-%, more preferably at least 95 weight-%, more preferably at least 99 weight-% of the at least one silicon-based inorganic compound are present in amorphous form. More preferably, at least 99.5 weight-%, more preferably at least 99.9 weight-%, more preferably at least 99.99 weight-% of the at least one silicon-based inorganic compound are present in amorphous form.

The solid dispersion according to the present invention can also comprise HPMC and at least one silicon-based inorganic compound.

The solid dispersion according to the present invention can also comprise at least one hydrophilic, organic polymer and at least one specific silicon-based inorganic adsorbent.

### First preferred embodiment

According to a first preferred embodiment of the present invention, the at least one pharmaceutically acceptable excipient used as matrix component is at least one organic polymer, wherein the organic polymer is hydroxypropylmethylcellulose (HPMC).

Therefore, the present invention also relates to a solid dispersion comprising amorphous brigatinib according to formula (A), and at least one pharmaceutically acceptable excipient, wherein at least 99 weight-% of the brigatinib comprised in the solid dispersion are present in amorphous form, at least 99 weight-% of the solid dispersion consist of the brigatinib and the at least one pharmaceutically acceptable excipient, and wherein the at least one pharmaceutically acceptable excipient is HPMC.

Preferably, the average molecular weight (M_{w}) of the hydroxypropylmethylcellulose, is in the range of from 7 to 225 kDa, more preferably in the range of from 7 to 100 kDa, more preferably in the range of from 7 to 30 kDa. According to the present invention, it is possible that the solid composition contains two or more hydroxypropylmethylcelluloses, which differ only in the average molecular weight M_{w}.

Preferably, the degree of substitution of methoxy groups (DS_{M}) of the hydroxypropylmethylcellulose is in the range of from 0.3 to 2.8, more preferably in the range of from 0.6 to 2.5, more preferably in the range of from 1.0 to 2.3, more preferably in the range of from 1.3 to 2.0. According to the present invention, it is possible that the solid composition contains two or more hydroxypropylmethylcelluloses, which differ only in the degree of substitution. The parameter DS_{M} describes the average number of methoxy substitutions per anhydroglucose unit of a given hydroxymethylcellulose.

Further according to the present invention, it is possible that the solid dispersion contains two or more hydroxypropylmethylcelluloses, which differ in the degree of substitution of methoxy groups and the average molecular weight M_{w}.

Chemical derivatives of hydroxypropylmethylcellulose, such as hydroxypropylmethylcellulose acetate succinate (HPMC-AS), are not hydroxypropylmethylcelluloses of the present invention.

### Second preferred embodiment

Therefore, according to a second preferred embodiment of the present invention, the at least one pharmaceutically acceptable excipient used as matrix component is a silicon-based inorganic compound preferably having a pH of at least 5.5 as determined using a pH meter in a solution of 400 mg of the silicon-based inorganic compound in 10 mL of de-ionized water at 25 °C, preferably consists of one silicon-based inorganic compound.

Therefore, the present invention also relates to a solid dispersion comprising amorphous brigatinib according to formula (A) and at least one pharmaceutically acceptable excipient, wherein the at least one pharmaceutically acceptable excipient is a silicon-based inorganic compound having a pH of at least 5.5 as determined using a pH meter in a solution of 400 mg of the silicon-based inorganic compound in 10 mL of de-ionized water at 25 °C, and wherein at least 99 weight-% of the brigatinib comprised in the solid dispersion are present in amorphous form, at least 99 weight-% of the solid dispersion consist of the brigatinib and the at least one silicon-based inorganic compound.

In particular the present invention also relates to a solid composition comprising amorphous brigatinib according to formula (A) and at least one pharmaceutically acceptable excipient, wherein the at least one pharmaceutically acceptable excipient is a silicon-based inorganic compound, and wherein at least 99 weight-% of the brigatinib comprised in the solid dispersion are present in amorphous form, at least 99 weight-% of the solid dispersion consist of the brigatinib and the at least one silicon-based inorganic compound, and wherein the pH of the silicon-based inorganic compound is in the range of from 5.5 to 9.0, preferably in the range of from 6.0 to 8.5, more preferably in the range of from 6.5 to 8.0.

Preferably, the bulk density of the at least one silicon-based inorganic compound is in the range of from 10 to 600 g/L, preferably in the range of from 30 to 450 g/L, more preferably in the range of from 50 to 400 g/L, such as from 50 g/L to 250 g/L. Generally, it is conceivable that the solid dispersion of the present invention contains at least one silicon-based inorganic compound having a bulk density in the above-defined preferred ranges and at least one silicon-based inorganic compound having a bulk density outside these ranges. Preferably, all silicon-based inorganic compounds comprised in the solid composition of the present invention have a bulk density in the above-defined preferred ranges.

In one aspect, the silicon-based inorganic compound is silica. The silica is preferably selected from the group consisting of fumed silica, precipitated silica, gel silica, colloidal silica, and a combination of two or more thereof, such as a combination of fumed silica and precipitated silica or a combination of fumed silica and colloidal silica or a combination of fumed silica and gel silica or a combination of precipitated silica and gel silica or a combination of precipitated silica and colloidal silica or a combination of gel silica and colloidal silica or a combination of fumed silica and precipitated silica and gel silica or a combination or fumed silica and gel silica and colloidal silica or a combination of precipitated silica and gel silica and colloidal silica or a combination of fumed silica and precipitated silica and gel silica and colloidal silica. Preferred silica include, but are not restricted to, the commercially available compounds Syloid^{®} 72 FP, Syloid^{®} 244 FP, both from Grace.

In another aspect, the silicon-based compound is a silicate. The silicate is preferably an aluminosilicate which, more preferably, additionally contains at least one alkali metal element selected from the group consisting of Li, Na, K, Rb, Cs and a combination of two or more thereof, preferably from the group consisting of Li, Na, K, and a combination of two or more thereof, more preferably from the group consisting of Na, K, and a combination of two or more thereof, and/or at least one alkaline earth metal element selected from the group consisting of Mg, Ca, Sr, Ba, and a combination of two or more thereof, preferably from the group consisting of Mg, Ca, Ba, and a combination of two or more thereof, preferably from the group consisting of Mg, Ca, and a combination of two or more thereof. More preferably, the silicate is an aluminosilicate which additionally contains at least one alkaline earth metal element selected from the group consisting of Mg, Ca, Sr, Ba, and a combination of two or more thereof, preferably from the group consisting of Mg, Ca, Ba, and a combination of two or more thereof, preferably from the group consisting of Mg, Ca, and a combination of two or more thereof. More preferably, the silicate is an aluminosilicate, which additionally contains Mg. Preferred silicates include, but are not restricted to, the commercially available compounds Neusilin^{®} UFL2, Neusilin^{®} US2, both from Fuji Chemical Industry Co., Ltd.

Therefore, the present invention also relates to the solid dispersion as described above, wherein the at least one silicon-based inorganic compound is selected from the group consisting of silica, silicates, and a combination of two or more thereof, wherein the silica is preferably selected from the group consisting of fumed silica, precipitated silica, gel silica, colloidal silica, and a combination of two or more thereof, and wherein the silicates are preferably aluminosilicates, preferably comprising at least one alkali metal element and/or at least one alkaline earth metal element, more preferably at least one alkaline earth metal element, more preferably magnesium.

Generally, the silica and/or the silicate can be present in crystalline or amorphous form. Preferably, at least 90 weight-%, more preferably at least 95 weight-%, more preferably at least 99 weight-% of the at least one silicon-based inorganic compound are present in amorphous form. More preferably, at least 99.5 weight-%, more preferably at least 99.9 weight-%, more preferably at least 99.99 weight-% of the at least one silicon-based inorganic compound are present in amorphous form.

### Preparation process of the solid dispersion

As described above, the present invention also relates to a process for preparing a solid dispersion comprising amorphous brigatinib and at least one pharmaceutically acceptable excipient, wherein the at least one pharmaceutically acceptable excipient is (i) hydroxypropylmethylcellulose, and/or (ii) a specific silicon-based inorganic adsorbent, the process comprising:
a) providing brigatinib;
b) dissolving or dispersing brigatinib provided in (a) and the at least one pharmaceutically acceptable excipient, wherein the at least one pharmaceutically acceptable excipient is (i) HPMC, and/or (ii) a silicon-based inorganic adsorbent preferably having a pH of at least 5.5 as determined using a pH meter in a solution of 400 mg of the silicon-based inorganic compound in 10 mL of de-ionized water at 25 °C, in a solvent to form a mixture; and
c) removing at least part, preferably essentially all, of the solvent to provide the solid dispersion.

### Step a)

Brigatinib may be provided by any method known to the person skilled in the art, such as, e.g., the methods described in WO2016/065028.

Brigatinib may be provided in any form, such as in crystalline, in amorphous form, or in a mixture of thereof. Brigatinib may be present in crystalline form, e.g. in form A or form B or form C or form D or as a mixture of two or more thereof. Alternatively, brigatinib in step (a) may be provided in amorphous form, for example according to Comparative Example 1 of the present invention. Brigatinib may also be provided as a crystalline solvate reported in described in WO2016/065028.

Thus, the present invention also relates to a process for preparing a solid dispersion and a solid dispersion obtained or obtainable by the above-described method, wherein in step (a) crystalline brigatinib, preferably crystalline brigatinib in polymorphic form A, B, C or D, or a mixture of two or more of these crystalline forms, more preferably crystalline brigatinib in polymorphic form A or B or as a mixture of thereof, is provided.

Alternatively, brigatinib in step (a) is provided in amorphous form.

### Step (b)

In step (b), brigatinib is preferably dissolved or dispersed together with the at least one pharmaceutically acceptable excipient as matrix compound in a suitable solvent. As excipient (i) a hydroxypropylmethylcellulose, or (ii) a silicon-based inorganic adsorbent preferably having a pH of at least 5.5 as determined using a pH meter in a solution of 400 mg of the silicon-based inorganic compound in 10 mL of de-ionized water at 25 °C can be used. Both components may be dissolved or dispersed together or subsequently. As excipient a mixture of (i) a hydroxypropylmethylcellulose and (ii) a silicon-based inorganic adsorbent can also be used. The term "a suitable solvent" as used herein refers to a solvent or solvent mixture in which both brigatinib and the at least one pharmaceutically acceptable excipient have adequate solubility or may be suitably dispersed. The term "adequate solubility" is denoted to mean a solubility at room temperature of brigatinib of greater than about 10 mg/ml.

In the case where the pharmaceutically acceptable excipient is HPMC, brigatinib and HPMC may require different solvents to obtain the desired solubility. A mixture of solvents can then be used. In this case, brigatinib may be dissolved in at least one solvent to give a mixture comprising the at least one solvent and brigatinib. Likewise, HPMC may be dissolved in at least one further solvent to give a mixture comprising HPMC and the at least one further solvent. Both mixtures may then be mixed together.

Brigatinib and HPMC may be dissolved or dispersed, together or subsequently, in the suitable solvent (including solvent mixtures).

Preferably, brigatinib is dissolved in at least one solvent, yielding a mixture comprising the at least one solvent and brigatinib, and HPMC is dissolved in at least one further solvent provided that the at least one further solvent is miscible with the at least one solvent used to dissolve brigatinib, yielding a mixture comprising HPMC and the at least one further solvent. Both mixtures are then mixed together to give a homogeneous mixture comprising brigatinib, HPMC and miscible solvents.

Examples of solvents (including solvent mixtures) include, but are not limited to, water, acetonitrile, C3-C5 ketones, C1-C2 halogenated hydrocarbons, C3-C4 alcohols, C2-C6 ethers, C3-C5 esters, and a combination of two or more thereof. More preferably, the solvent comprises, and for example consists of, dichloromethane, chloroform, ethanol, methanol, THF, methyl THF, 2-propanol, ethyl acetate, acetone, acetonitrile, water or mixtures of two or more thereof. Even more preferably, the solvent comprises, and for example consists of, dichloromethane, THF, methyl THF, acetone, ethanol, acetonitrile, water or mixtures of two or more thereof. More preferably, the solvent comprises acetone, ethanol, acetonitrile, water or mixtures of two or more thereof. Most preferably the solvent comprises, more preferably the solvent is, acetonitrile, water or mixtures of acetonitrile and water.

The solution obtained in step (b) may be directly used in step (c) of the method according to the invention. The solution formed may also be purified before used in step (c). The term "purified" in this context means e.g. that non-dissolved particles, such as non-dissolved HPMC and/or non-dissolved brigatinib, may be removed by suitable methods known to those skilled in the art such as centrifugation, filtration, ultrafiltration or the like.

Thus, the present invention also relates to a process, as described above, the process comprising
(a) providing brigatinib,
(b) dissolving brigatinib provided in step (a) and HPMC in a solvent to form a solution, then optionally filtrating the solution, and
(c) removing at least part, preferably essentially all, of the solvent to give the solid dispersion.

Regarding the weight ratio of brigatinib and the hydroxypropylmethylcellulose relative to the at least one solvent, no specific restrictions exist provided that the finally obtained mixture is a solution wherein the hydroxypropylmethylcellulose and brigatinib are dissolved in the at least one solvent, which solution can be subjected to the subsequent step (c). Preferably, the weight ratio of brigatinib plus the hydroxypropylmethylcellulose relative to the at least one solvent, preferably acetone, ethanol, acetonitrile, water or mixtures of two or more thereof, even more preferably acetonitrile, water or mixtures of acetonitrile and water, is in the range of from 0.01 : 1 to 0.4 : 1, preferably in the range of from 0.02 : 1 to 0.3 : 1, more preferably in the range of from 0.05 : 1 to 0.3 : 1.

To accelerate and/or improve the dissolution process of brigatinib in the at least one solvent, suitable methods can be applied. For example, the dissolution process can be influenced by choosing suitable temperatures, by stirring, and/or by subjecting the respective mixtures to sonication, wherein these methods can be applied during the entire or one or more parts of the mixing process.

Preferably, the mixture of brigatinib and the at least one hydroxypropylmethylcellulose in the at least one solvent, is prepared at a temperature in the range of from 10 to 40 °C, more preferably in the range of from 15 to 35 °C, more preferably in the range of from 20 to 30 °C, preferably at ambient pressure.

In the case where pharmaceutically acceptable excipient is a silicon-based inorganic compound, it is preferred that the process comprises dispersing the at least one silicon-based inorganic compound in a solution comprising the dissolved brigatinib.

Consequently, solvents are preferred in which brigatinib can be dissolved and the at least one silicon-based inorganic compound can be dispersed. Preferably, the at least one solvent is selected from the group consisting of water, acetonitrile, C3-C5 ketones, C1-C2 halogenated hydrocarbons, C3-C4 alcohols, C2-C6 ethers, C3-C5 esters, and a combination of two or more thereof. More preferably, the solvent comprises, and for example consists of, dichloromethane, chloroform, ethanol, methanol, THF, methyl THF, 2-propanol, ethyl acetate, acetone, acetonitrile, water or mixtures of two or more thereof. Even more preferably, the solvent comprises, and for example consists of, dichloromethane, THF, methyl THF, acetone, ethanol, acetonitrile, water or mixtures of two or more thereof. More preferably, the solvent comprises acetone, ethanol, acetonitrile, water or mixtures of two or more thereof. Most preferably the solvent comprises, more preferably the solvent is acetonitrile, water or mixtures of acetonitrile and water.

Regarding the weight ratio of brigatinib and the at least one silicon-based inorganic compound relative to the at least one solvent, no specific restrictions exist provided that the finally obtained mixture is a mixture wherein the at least one silicon-based inorganic compound is dispersed in a solution of the brigatinib in the at least one solvent, which mixture can be subjected to the subsequent step (c). Preferably, the weight ratio of brigatinib plus the at least one silicon-based inorganic compound, preferably brigatinib plus the at least one silica, relative to the at least one solvent, preferably dichloromethane, acetone, ethanol, acetonitrile, water or mixtures of two or more thereof, even more preferably acetonitrile, water or mixtures of acetonitrile and water, is in the range of from 0.01 : 1 to 0.4 : 1, preferably in the range of from 0.02 : 1 to 0.3 : 1, more preferably in the range of from 0.05 : 1 to 0.3 : 1. Also preferably, the weight ratio of brigatinib plus the at least one silicon-based inorganic compound, preferably brigatinib plus the at least one silicate, preferably the aluminosilicates preferably comprising at least one alkali metal element and/or at least one alkaline earth metal element, more preferably at least one alkaline earth metal element, more preferably magnesium, relative to the at least one solvent, preferably acetone, ethanol, acetonitrile, water or mixtures of two or more thereof, even more preferably acetonitrile, water or mixtures of acetonitrile and water, is in the range of from 0.01 : 1 to 0.4 : 1, preferably in the range of from 0.02 : 1 to 0.3 : 1, more preferably in the range of from 0.05 : 1 to 0.3 : 1.

To accelerate and/or improve the dissolution process of brigatinib in the at least one solvent, suitable methods can be applied. For example, the dissolution process can be influenced by choosing suitable temperatures, by stirring, and/or by subjecting the respective mixtures to sonication, wherein these methods can be applied during the entire or one or more parts of the mixing process.

Preferably, the dispersion of the at least one silicon-based inorganic compound, preferably selected from the group consisting of silica, silicates, and a combination of two or more thereof, in the solution of brigatinib in the at least one solvent, is prepared at a temperature in the range of from 10 to 40 °C, more preferably in the range of from 15 to 35 °C, more preferably in the range of from 20 to 30 °C, preferably at ambient pressure.

### Step (c)

In step (c) of the above described method, at least part, preferably essentially all, of the solvent is removed. "Essentially all" is denoted to mean that at least 95 % by weight, more preferably at least 96 % by weight, more preferably at least 97 % by weight, more preferably at least 98 % by weight, more preferably at least 99 % by weight, such as 99.5% or even more preferably all of the solvent present in the solution or suspension according to step (b) is removed in step (c).

The removal of the solvent may be carried out by any suitable method known to those skilled in the art such as evaporation, spray drying, lyophilization, melt extrusion, drum drying, freeze-drying or other solvent removal processes. Preferably, the solvent is removed by spray drying, lyophilization or evaporation. Spray drying is a process well known to those skilled in the art for preparing solid dispersions. In such a spray drying process, the solution is pumped through an atomizer into a drying chamber thereby removing the solvent to form the solid dispersion. A drying process uses hot gases, such as air, nitrogen, nitrogen-enriched air or argon, to dry the particles. The solution can be atomized by conventional means well known in the art, such as a two-fluid sonication nozzle and a two-fluid non-sonication nozzle.

If the solvent is removed by lyophilization, the sample temperature during lyophilization may be varied or held essentially constant and is preferably in the range of from 20 to 40°C, more preferably in the range of from 25 to 40°C.

### Pharmaceutical composition

In another aspect, the present invention relates to the use of the solid dispersion as described above or a solid dispersion obtained or obtainable by the above-described method, comprising amorphous brigatinib and at least one pharmaceutically acceptable excipient, wherein the at least one pharmaceutically acceptable excipient is (i) hydroxypropylmethylcellulose, and/or (ii) a silicon-based inorganic adsorbent as defined herein, for the preparation of a pharmaceutical composition.

In other words, the solid dispersion of the present invention is a useful intermediate in the preparation of a pharmaceutical composition comprising amorphous brigatinib.

In a further aspect, the present invention therefore relates to a pharmaceutical composition comprising the solid dispersion of the present invention and one or more additional pharmaceutically acceptable excipient(s).

In a preferred embodiment, the predetermined and/or effective amount of brigatinib in the pharmaceutical composition comprising the solid dispersion of the present invention is from 5 mg to 200 mg, for example selected from the group consisting of 5 mg, 10 mg, 15 mg, 30 mg, mg, 45 mg, 60 mg, 80 mg, 90 mg, 100 mg, 140 mg and 180 mg.

The pharmaceutical composition of the present invention is preferably an oral solid dosage form, such as a tablet.

The additional excipient of the pharmaceutical composition of the present invention can be selected from diluents, glidants, lubricants, surfactants and disintegrants. Reference is made to the extensive literature on the subject for these and other excipients and procedures mentioned herein, see in particular Handbook of Pharmaceutical Excipients, Third Edition, edited by Arthur H. Kibbe, American Pharmaceutical Association, Washington, USA and Pharmaceutical Press, London; and Lexikon der Hilfsstoffe für Pharmazie, Kosmetik and angrenzende Gebiete edited by H.P. Fiedler, 4th Edition, Edito Cantor, Aulendorf and earlier editions which are incorporated herein by reference.

In a preferred embodiment the pharmaceutical composition of the present invention is an oral solid dosage form, such as a tablet, and comprises as further excipients a diluent, a disintegrant and a lubricant, preferably further comprising a glidant, even more preferably further comprising a glidant and a surfactant.

Examples of diluents are microcrystalline cellulose, calcium phosphate, calcium carbonate, starch, spray-dried lactose, anhydrous lactose, lactose monohydrate and mannitol. Spray dried lactose is a preferred diluent.

Examples of disintegrants include but are not restricted to maize starch; CMC-Ca; CMC-Na; microcrystalline cellulose; cross-linked PVP, e.g. as known and commercially available under the trade names Crospovidone^{®}, Polyplasdone^{®}, available commercially from the ISP company, or Kollidon^{®} XL; alginic acid; sodium alginate; and guar gum. Cross-linked PVP, e.g. Crospovidone^{®} is a preferred disintegrant.

Examples of glidants include one or more of the following: silica, colloidal silica, e.g. colloidal silica anhydrous, e.g. Aerosil^{®} 200, magnesium trisilicat, powdered cellulose, starch and talc. Colloidal silica anhydrous or/and colloidal silicon dioxide are preferred glidants.

Examples of lubricants include one or more of the following: Mg-, Al- or Ca-stearate, PEG 4000 - 8000 and/or talc. Magnesium stearate is a preferred lubricant.

Examples of surfactants include one or more of the following: an alkyl sulfate salt, such as sodium laurylsulfate, or a poloxamer. Sodium lauryl sulfate is a preferred surfactant.

It will be appreciated that any given excipient may serve more than one function e.g. as disintegrant, binder, glidant, and/or lubricant.

Below are two examples of pharmaceutical compositions of the present invention:

| | Example 1 [mg] | Example 2 [mg] |
|---|---|---|
| ASD Brigatinib /Syloid^{®} 72 FP 1:1 | 0,060 | |
| ASD Brigatinib /HPMC/Syloid^{®} 72 FP 1:0.5:0.5 | | 0,060 |
| lactose monohydrate | 0,020 | 0,020 |
| microcrystalline cellulose | 0,061 | 0,061 |
| sodium starch glycolate (Type A) | 0,004 | 0,004 |
| magnesium stearate | 0,003 | 0,003 |
| colloidal silica | 0,002 | 0,002 |
| = Mass of tablet core | 0,150 | 0,150 |
| + Opadry II | 0,005 | 0,005 |
| = Mass of film coated tablet | 0,155 | 0,155 |

The oral solid dosage form, and preferably the tablet, of the present invention may be slightly susceptible to high humidity. It is therefore preferred that the oral dosage forms are packaged in a packaging material having a comparatively low water vapour transmission rate of at most 1 g m⁻² d⁻¹, more preferably of at most 0.4 g m⁻² d⁻¹, as measured according to standard DIN 53122-1. An example of such a packaging material is an Alu/Alu blister, but also plastic-based packaging material meeting the above requirements are available.

The following non-limiting examples are illustrative for the disclosure.

### EXAMPLES

The following analytical method and parameters have been applied for the generation of the powder X-ray data disclosed in the present invention:

### Powder X-ray diffraction

Powder X-ray diffraction was performed with a PANalytical X'Pert PRO diffractometer equipped with a theta/theta coupled goniometer in transmission geometry, Cu-Kalpha_{1,2} radiation (wavelength 0.15419 nm) with a focusing mirror and a solid state PIXcel detector. Diffractograms were recorded at a tube voltage of 45 kV and a tube current of 40 mA, applying a stepsize of 0.013° 2-Theta with 40s per step (255 channels) in the angular range of 2° to 40° 2-Theta at ambient conditions.

### Determination of the moisture stability

Moisture stability at accelerated stress conditions was performed in a Memmert constant climate chamber HPP110. 30-50 mg of a given solid composition prepared according to the Examples and Comparative Example herein were exposed to an atmosphere having a relative humidity of 75±0.5 % and a temperature of 40±0.5 °C for a period of time as indicated in Tables 1-3 below and analyzed via PXRD as described above.

### Thermogravimetric mass spectroscopy (TGMS)

Thermogravimetric analysis (TGA) was performed on a Mettler Toledo TGA/DSC 1 instrument. Samples were heated in 100 µl aluminium pans closed with aluminium lids. Lids were automatically pierced at the beginning of the measurement. Samples were initially kept at 25°C for 2.5 minutes and then heated from room temperature to 250°C at a rate of 10°C/min. Nitrogen (purge rate 30 ml/min) was used as purge gas.

Mass spectrometry (MS) measurements, coupled with thermogravimetric analysis (TGA), were performed with a Pfeiffer GSD 320 ThermoStar Gas Analysis System. A part of the headspace was evacuated with a capillary placed near the TG sample pan, and mass fragments between 1 - 100 atomic mass units (amu) were analyzed simultaneously without further separation applying a secondary electron multiplier (SEM) for signal amplification. Since samples were kept at 25°C for 2.5 minutes and then heated with a heating rate of 10°C/min, the ordinate of mass trace printouts, displayed in minutes, can be directly related to temperatures applying a factor of 10 (e.g. 10 minutes correspond to 100°C in the TGA experiment).

### Comparative Example 1: Preparation of amorphous brigatinib and physical stability of amorphous brigatinib under accelerated stability conditions

Brigatinib (1.50 g, e.g. prepared according to the procedures disclosed in WO 2016/065028 A1, part V) was dissolved in 75 mL of acetonitrile/water solution (volume ratio 50:50). The solution was filtered. The homogeneous solution was frozen in a bath of liquid nitrogen and lyophilized at a pressure of from 0.02 to 1.0 mbar, yielding amorphous brigatinib, as confirmed by the PXRD depicted in Figure 1. The obtained amorphous brigatinib was stored open under accelerated stability conditions (temperature: 40°C, relative humidity: 75%). The material was analysed by powder X-ray diffraction after one and three weeks and the results are summarized in Table 1.

**Table 1: Results of stress tests with neat amorphous brigatinib (Form A = crystalline form A of WO 2016/065028 A1)**

| **Sample** | **Carrier** | **Physical form** | **PXRD analysis after storage at 40°C and 75% RH for** | |
|---|---|---|---|---|
| | | | **1 week** | **3 weeks** |
| Brigatinib | - | amorphous | Form A | Form A |

As it can be seen from Table 1 neat amorphous brigatinib is physically unstable and readily crystallizes during the stress test. After one week upon storage at a temperature of 40°C and a relative humidity of 75% sample contained crystalline Form A of brigatinib. PXRD pattern of the brigatinib sample after one week upon storage at a temperature of 40°C and a relative humidity of 75% is shown in Figure 2.

### Example 1: Preparation of amorphous solid dispersions comprising brigatinib and HPMC

Brigatinib (103 mg, e.g. prepared according to the procedures disclosed in WO 2016/065028 A1, part V) and 102 mg of hydroxypropylmethylcellulose (HPMC E5, having an average M_{w} of 10 000 g/mol; commercially available from Dow Chemical Co. under the trade name Methocel E5) were dissolved in 7.5 mL of acetonitrile/water solution (volume ratio 50:50). The homogeneous solution was frozen in a bath of liquid nitrogen and lyophilized at a pressure of from 0.02 to 1.0 mbar, yielding an amorphous solid dispersion of brigatinib. The resulting solid dispersion was subjected to a moisture stability test as described in Example part herein. The results are summarized in Table 2 below. Representative PXRD pattern of the solid dispersion after its preparation is shown in Figure 3, the PXRD pattern of the solid dispersion after 1 week upon storage at a temperature of 40 °C and a relative humidity of 75% is shown in Figure 4. Figures 3 and 4 show that the brigatinib comprised in the solid dispersion was in amorphous form and did not crystallize during the moisture stability test.

### Example 1b:

Brigatinib (103 mg, e.g. prepared according to the procedures disclosed in WO 2016/065028 A1, part V) was dissolved in 4.5 mL of acetonitrile/water solution (volume ratio 50:50). Hydroxypropylmethylcellulose (102 mg, HPMC E5, having an average Mw of 10 000 g/mol; commercially available from Dow Chemical Co. under the trade name Methocel E5) was dissolved in 1.5 mL water, followed by addition of 1.5 mL acetonitrile. Both solution were mixed together and the homogeneous solution was frozen in a bath of liquid nitrogen and lyophilized at a pressure of from 0.02 to 1.0 mbar, yielding an amorphous solid dispersion of brigatinib.

### Example 2: Preparation of amorphous solid dispersions comprising brigatinib and Syloid^{®} 72FP

### Example 2-1

Brigatinib (102 mg, e.g. prepared according to the procedures disclosed in WO 2016/065028 A1, part V) was dissolved in 5 mL of acetonitrile/water solution (volume ratio 50:50). The brigatinib solution was added to a suspension containing 103 mg of Syloid^{®} 72FP (synthetic amorphous silica commercialized by Grace) in 2 mL of acetonitrile/water solution (volume ratio 50:50) to obtain a solution of brigatinib with suspended Syloid^{®} 72FP. The suspension was homogeneously frozen in a bath of liquid nitrogen and lyophilized at a pressure of from 0.02 to 1.0 mbar, yielding an amorphous solid dispersion of brigatinib. The resulting solid dispersion was subjected to a moisture stability test as described in Example part herein. The results are summarized in Table 3 below. Representative PXRD pattern of the solid dispersion after its preparation is shown in Figure 5, the PXRD pattern of the solid dispersion after 1 week upon storage at a temperature of 40 °C and a relative humidity of 75% is shown in Figure 6. Figures 5 and 6 show that the brigatinib comprised in the solid dispersion was in amorphous form and did not crystallize during the moisture stability test.

### Example 2-2

To brigatinib (101 mg, e.g. prepared according to the procedures disclosed in WO 2016/065028 A1, part V) and 102 mg of Syloid^{®} 72FP (synthetic amorphous silica commercialized by Grace) were added 7 mL of acetonitrile/water solution (volume ratio 50:50) to obtain a solution of brigatinib with suspended Syloid^{®} 72FP. The suspension was homogeneously frozen in a bath of liquid nitrogen and lyophilized at a pressure of from 0.02 to 1.0 mbar, yielding an amorphous solid dispersion of brigatinib. The PXRD pattern of the solid dispersion after its preparation was essentially identical to the one shown in Figure 5.

### Example 3: Preparation of amorphous solid dispersions comprising brigatinib and Neusilin^{®} UFL2

### Example 3-1

Brigatinib (102 mg, e.g. prepared according to the procedures disclosed in WO 2016/065028 A1, part V) was dissolved in 5 mL of acetonitrile/water solution (volume ratio 50:50). The brigatinib solution was added to a suspension containing 103 mg of Neusilin^{®} UFL2 (neutral magnesium aluminometasilicate commercialized by Fuji Chemical Industry Col., Ltd.) in 2 mL of acetonitrile/water solution (volume ratio 50:50) to obtain a solution of brigatinib with suspended Neusilin^{®} UFL2. The suspension was homogeneously frozen in a bath of liquid nitrogen and lyophilized at a pressure of from 0.02 to 1.0 mbar, yielding an amorphous solid dispersion of brigatinib. The resulting solid dispersion was subjected to a moisture stability test as described in Example part herein. The results are summarized in Table 3 below. Representative PXRD pattern of the solid dispersion after its preparation is shown in Figure 7, the PXRD pattern of the solid dispersion after 1 week upon storage at a temperature of 40 °C and a relative humidity of 75% is shown in Figure 8. Figures 7 and 8 show that the brigatinib comprised in the solid dispersion was in amorphous form and did not crystallize during the moisture stability test.

### Example 3-2

To brigatinib (102 mg, e.g. prepared according to the procedures disclosed in WO 2016/065028 A1, part V) and 101 mg of Neusilin^{®} UFL2 (neutral magnesium aluminometasilicate commercialized by Fuji Chemical Industry Col., Ltd.) were added 7 mL of acetonitrile/water solution (volume ratio 50:50) to obtain a solution of brigatinib with suspended Neusilin^{®} UFL2. The suspension was homogeneously frozen in a bath of liquid nitrogen and lyophilized at a pressure of from 0.02 to 1.0 mbar, yielding an amorphous solid dispersion of brigatinib. The PXRD pattern of the solid dispersion after its preparation was essentially identical to the one shown in Figure 7.

### Example 4: Physical stability of the solid dispersions with brigatinib and organic polymers

Brigatinib (about 100 mg, e.g. prepared according to the procedures disclosed in WO 2016/065028 A1, part V) and the organic polymer (about 100 mg) according to Table 2 were dissolved in 7 to 8 mL of acetonitrile/water solution (volume ratio 50:50). The homogeneous solution was frozen in a bath of liquid nitrogen and lyophilized at a pressure of from 0.02 to 1.0 mbar, yielding an amorphous solid dispersion of brigatinib. The so obtained solid dispersion was open stored under accelerated stability conditions (temperature: 40°C, relative humidity: 75%) for three weeks. The sample was periodically analysed by powder X-ray diffraction and the results are summarized in Table 2.

**Table 2: Results of stress tests with solid dispersions of amorphous brigatinib and organic polymer (Form A = crystalline form A of WO 2016/065028 A1)**

| **Sample** | **Carrier** | **Physical form** | **PXRD analysis after storage at 40°C and 75% RH for** | |
|---|---|---|---|---|
| | | | **1 week** | **3 weeks** |
| A | HPMC | amorphous | amorphous | amorphous |
| B | PVP (40000) | amorphous | Form A + amorphous | Form A + amorphous |

In contrast to solid dispersions prepared with hydroxypropylmethylcellulose (HPMC), stabilization with polyvinylpyrrolidon (PVP 40000) was not as successful since crystalline phase was already visible in the one-week sample.

### Example 5: Physical stability of solid dispersions with amorphous brigatinib and various silicon-based inorganic adsorbents at accelerated stress conditions

To brigatinib (about 100 mg, e.g. prepared according to the procedures disclosed in WO 2016/065028 A1, part V) and silicon-based inorganic adsorbent (about 100 mg) according to Table 3 were added 7-8 mL of acetonitrile/water solution (volume ratio 50:50) to obtain a solution of brigatinib with suspended silicon-based inorganic adsorbent. The suspension was homogeneously frozen in a bath of liquid nitrogen and lyophilized at a pressure of from 0.02 to 1.0 mbar, yielding an amorphous solid dispersion of brigatinib. The so obtained solid dispersion was open stored under accelerated stability conditions (temperature: 40°C, relative humidity: 75%) for three weeks. The sample was periodically analysed by powder X-ray diffraction and the results are summarized in Table 3.

**Table 3: Results of stress tests with solid dispersions of amorphous brigatinib and silicon-based inorganic adsorbent (Form A = crystalline form A of WO 2016/065028 A1)**

| **Sample** | **Carrier** | **Physical form** | **PXRD analysis after storage at 40°C and 75% RH for** | |
|---|---|---|---|---|
| | | | **1 week** | **3 weeks** |
| A | Syloid^{®} 72FP | amorphous | amorphous | amorphous |
| B | Syloid^{®} AL-1FP | amorphous | amorphous + Form A | amorphous + Form A |
| C | Neusilin^{®} UFL2 | amorphous | amorphous | Amorphous (traces of Form A) |
| D | Neusilin^{®} US2 | amorphous | amorphous | amorphous (traces of Form A) |

Syloid^{®} AL-1 FP: synthetic amorphous silica commercialized by Grace (powder with a bulk density of 566 g/L; pH 5.1, pH value as provided by Grace in the product information sheet. This pH value corresponds well with the pH value determined by the present inventors by using a pH meter in a solution of 400 mg of the silicon-based inorganic compound in 10 mL of de-ionized water at 25 °C).

Syloid^{®} 72 FP: synthetic amorphous silica commercialized by Grace (powder with a bulk density of 112 g/L; pH 7.2, pH value as provided by Grace in the product information sheet.

This pH value corresponds well with the pH value determined by the present inventors by using a pH meter in a solution of 400 mg of the silicon-based inorganic compound in 10 mL of de-ionized water at 25 °C).

Neusilin UFL2^{®}: neutral magnesium aluminometasilicate commercialized by Fuji Chemical Industry Col., Ltd. (powder with a tapped bulk density of 100-170 g/L; pH 6.0 - 8.0, pH range as provided by Fuji Chemical Industry Col., Ltd. in the product information sheet. This pH range corresponds well with the pH value determined by the present inventors by using a pH meter in a solution of 400 mg of the silicon-based inorganic compound in 10 mL of de-ionized water at 25 °C).

Neusilin US2^{®}: neutral magnesium aluminometasilicate commercialized by Fuji Chemical Industry Col., Ltd (granule with a tapped bulk density of 160-220 g/L; pH 6.0 - 8.0, pH range as provided by Fuji Chemical Industry Col., Ltd. in the product information sheet. This pH range corresponds well with the pH value determined by the present inventors by using a pH meter in a solution of 400 mg of the silicon-based inorganic compound in 10 mL of de-ionized water at 25 °C).

## Claims

1. A solid dispersion comprising amorphous 5-chloro-*N*4-[2-(dimethylphosphoryl)phenyl]-*N*2-[2-methoxy-4-[4-(4-methylpiperazin-1-yl)piperidin-1-yl]phenyl]pyrimidine-2,4-diamine (brigatinib) and at least one pharmaceutically acceptable excipient, wherein the at least one pharmaceutically acceptable excipient is a silicon-based inorganic adsorbent having a BET specific surface area of at least 1 m²/g and wherein the silicon-based inorganic compound is **characterized by** having a pH of at least 5.5 as determined using a pH meter in a solution of 400 mg of the silicon-based inorganic compound in 10 mL of de-ionized water at 25 °C.

2. The solid dispersion according to claim 1, wherein crystalline brigatinib is not present.

3. The solid dispersion according to any one of claims 1 to 2, wherein the solid dispersion is amorphous.

4. The solid dispersion according to any one of claims 1 to 3, wherein the pharmaceutically acceptable excipient is a silicon-based inorganic compound selected from silica and aluminosilicates.

5. The solid dispersion according to any one of the preceding claims, wherein the weight ratio of brigatinib and the pharmaceutically acceptable excipient is from 1.0: 0.5 to 1.0 : 10.0, and wherein the weight ratio is calculated based on the total amount of brigatinib present in said solid dispersion and on the total amount of the pharmaceutically acceptable excipients present in said solid dispersion.

6. A pharmaceutical composition comprising a solid dispersion according to any one of claims 1 to 5, and further comprising one or more additional pharmaceutically acceptable excipients.

7. A process for the preparation of the solid dispersion according to any one of claims 1 to 5 comprising:
a) providing brigatinib;
b) dissolving or dispersing brigatinib provided in step (a) and the silicon-based inorganic adsorbent,
in a solvent to form a mixture, and
c) removing at least part, preferably essentially all, of the solvent to provide the solid dispersion.

8. The process according to claim 7, wherein the at least one pharmaceutically acceptable excipient is a silicon-based inorganic adsorbent and wherein step b) results in a suspension.

## Patentansprüche

1. Feste Dispersion, umfassend amorphes 5-Chlor-N4-[2-(dimethylphosphoryl)phenyl]-N2-[2-Methoxy-4-[4-(4-methylpiperazin-1-yl)piperidin-1-yl]phenyl]pyrimidin-2,4-diamin (Brigatinib) und mindestens einen pharmazeutisch akzeptablen Hilfsstoff, wobei der mindestens eine pharmazeutisch akzeptable Hilfsstoff ein anorganisches Adsorptionsmittel auf Siliziumbasis mit einer spezifischen BET-Oberfläche von mindestens 1 m²/g ist und wobei die anorganische Verbindung auf Siliziumbasis **dadurch gekennzeichnet ist, dass** sie einen pH-Wert von mindestens 5,5 aufweist, bestimmt mit einem pH-Meter in einer Lösung von 400 mg der anorganischen Verbindung auf Siliciumbasis in 10 ml entionisiertem Wasser bei 25 °C.

2. Feste Dispersion nach Anspruch 1, wobei kein kristallines Brigatinib vorhanden ist.

3. Feste Dispersion nach einem der Ansprüche 1 bis 2, wobei die feste Dispersion amorph ist.

4. Feste Dispersion nach einem der Ansprüche 1 bis 3, wobei der pharmazeutisch akzeptable Hilfsstoff eine anorganische Verbindung auf Siliciumbasis ist, die aus Siliciumdioxid und Alumosilicaten ausgewählt ist.

5. Feste Dispersion nach einem der vorhergehenden Ansprüche, wobei das Gewichtsverhältnis von Brigatinib und dem pharmazeutisch akzeptablen Hilfsstoff 1,0 : 0,5 bis 1,0 : 10,0 beträgt und wobei das Gewichtsverhältnis auf der Grundlage der Gesamtmenge an Brigatinib, die in der festen Dispersion vorhanden ist, und auf der Gesamtmenge der pharmazeutisch akzeptablen Hilfsstoffe, die in der festen Dispersion vorhanden sind, berechnet wird.

6. Pharmazeutische Zusammensetzung, umfassend eine feste Dispersion nach irgendeinem der Ansprüche 1 bis 5 und ferner umfassend einen oder mehrere zusätzliche pharmazeutisch akzeptable Hilfsstoffe.

7. Verfahren zur Herstellung der festen Dispersion nach irgendeinem der Ansprüche 1 bis 5, umfassend:
a) Bereitstellen von Brigatinib;
b) Lösen oder Dispergieren von Brigatinib, das in Schritt (a) bereitgestellt wurde, und des anorganischen Adsorptionsmittels auf Siliziumbasis,
in einem Lösungsmittel, um eine Mischung zu bilden, und
c) Entfernen mindestens eines Teils, vorzugsweise im Wesentlichen des gesamten Lösungsmittels, um die feste Dispersion bereitzustellen.

8. Verfahren nach Anspruch 7, wobei der mindestens eine pharmazeutisch akzeptable Hilfsstoff ein anorganisches Adsorptionsmittel auf Siliziumbasis ist und wobei Schritt b) zu einer Suspension führt.

## Revendications

1. Dispersion solide comprenant du 5-chloro-*N*4-[2-(diméthylphosphoryl)phényl]-*N*2-[2-méthoxy-4-[4-(4-méthylpipérazin-1-yl)pipéridin-1-yl]phényl]pyrimidine-2,4-diamine (brigatinib) amorphe et au moins un excipient pharmaceutiquement acceptable, dans laquelle le au moins un excipient pharmaceutiquement acceptable est un adsorbant inorganique à base de silicium présentant une surface spécifique BET d'au moins 1 m²Jg et dans laquelle le composé inorganique à base de silicium est **caractérisé en ce qu'**il présente un pH d'au moins 5,5 tel que déterminé en utilisant un pH-mètre dans une solution de 400 mg du composé inorganique à base de silicium dans 10 ml d'eau désionisée à 25 °C.

2. Dispersion solide selon la revendication 1, dans laquelle du brigatinib cristallin n'est pas présent.

3. Dispersion solide selon l'une quelconque des revendications 1 à 2, dans laquelle la dispersion solide est amorphe.

4. Dispersion solide selon l'une quelconque des revendications 1 à 3, dans laquelle l'excipient pharmaceutiquement acceptable est un composé inorganique à base de silicium sélectionné parmi la silice et les aluminosilicates.

5. Dispersion solide selon l'une quelconque des revendications précédentes, dans laquelle le rapport pondéral du brigatinib et de l'excipient pharmaceutiquement acceptable est de 1,0 : 0,5 à 1,0 : 10,0, et dans laquelle le rapport pondéral est calculé sur la base de la quantité totale de brigatinib présent dans ladite dispersion solide et de la quantité totale des excipients pharmaceutiquement acceptables présents dans ladite dispersion solide.

6. Composition pharmaceutique comprenant une dispersion solide selon l'une quelconque des revendications 1 à 5, et comprenant en outre un ou plusieurs excipients pharmaceutiquement acceptables supplémentaires.

7. Processus pour la préparation de la dispersion solide selon l'une quelconque des revendications 1 à 5, comprenant les étapes consistant à :
a) fournir du brigatinib ;
b) dissoudre ou disperser du brigatinib fourni à l'étape (a) et l'adsorbant inorganique à base de silicium,
dans un solvant pour former un mélange, et
c) retirer au moins une partie, de préférence sensiblement la totalité, du solvant pour fournir la dispersion solide.

8. Processus selon la revendication 7, dans lequel le au moins un excipient pharmaceutiquement acceptable est un adsorbant inorganique à base de silicium et dans lequel l'étape b) donne lieu à une suspension.
